(19) Europäisches Patentamt
European Patent Office
Office européen des brevets

(11) Numéro de publication : **0 481 853 A1**

(12) # DEMANDE DE BREVET EUROPEEN

(21) Numéro de dépôt : **91402716.4**

(22) Date de dépôt : **11.10.91**

(51) Int. Cl.[5] : **C07D 401/06,** A61K 31/47

(30) Priorité : **18.10.90 FR 9012872**

(43) Date de publication de la demande : **22.04.92 Bulletin 92/17**

(84) Etats contractants désignés : **AT BE CH DE DK ES FR GB GR IT LI LU NL SE**


(71) Demandeur : **SYNTHELABO**
**58 rue de la Glacière**
**F-75013 Paris (FR)**

(72) Inventeur : **Frost, Jonathan**
**23, Route de Montjean**
**F-91320 Wissous (FR)**
Inventeur : **Lardenois, Patrick**
**18, Rue de Varengue**
**F-92340 Bourg la Reine (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**


(54) **1-(3,4-Dihydro-2-oxo-1H-quinoléin-6-yl)-2-[4-(2-phényléthyl)-pipéridin-1-yl]éthanol et-éthane, leur préparation et leur application en thérapeutique.**


(57) Composés de formule générale (I)

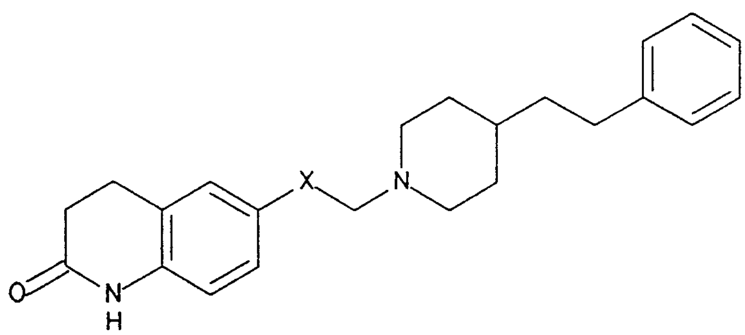

dans laquelle X représente un groupe de formule CO ou CHOH, à l'état de bases libres ou de sels d'addition à des acides et, lorsque X représente un groupe de formule CHOH, sous forme d'énantiomères purs ou de mélanges d'énantiomères.
Application en thérapeutique.


EP 0 481 853 A1

La présente invention a pour objet le 1-(3,4-dihydro-2-oxo-1*H*-quinoléin-6-yl)-2-[4-(2-phényléthyl)pipéridin-1-yl]éthanol et la 1-(3,4-dihydro-2-oxo-1*H*-quinoléin-6-yl)-2-[4-(2-phényléthyl)pipéridin-1-yl]éthanone, leur préparation et leur application en thérapeutique.

La demande de brevet GB-2071094 contient une formule chimique générale qui englobe de très nombreux composés, et en particulier les composés de l'invention. Toutefois cette demande ne décrit pas ces derniers, ni d'ailleurs aucun dérivé analogue comportant un groupe [4-(2-phényléthyl)pipéridin-1-yle]. Les composés de l'invention sont donc à considérer comme nouveaux.

Par ailleurs la demande de brevet citée énumère un certain nombre d'utilisations thérapeutiques des composés qu'elle décrit, utilisations parmi lesquelles ne figurent pas celles de l'invention.

Les composés de l'invention répondent à la formule générale (I)

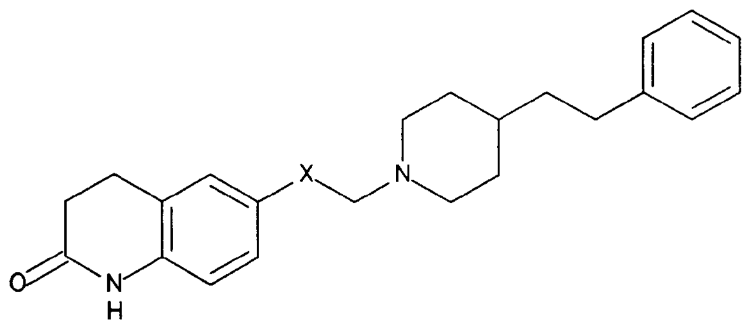

dans laquelle X représente un groupe de formule CO ou CHOH. Dans ce dernier cas, l'atome de carbone de ce groupe est chiral, de sorte que le composé correspondant peut exister sous forme d'énantiomères purs ou de mélanges d'énantiomères. Les composés peuvent encore exister à l'état de base libre ou de sels d'addition à des acides.

Ces diverses formes font bien entendu partie de l'invention.

Les composés de l'invention peuvent être obtenus par réaction de la 6-(2-chloro-1-oxoéthyl)-3,4-dihydro-1*H*-quinoléin-2-one (décrite dans la demande de brevet citée) avec la 4-(2-phényléthyl)pipéridine (décrite dans C.A., **52**, 8138a (1958)). On obtient d'abord la cétone de formule générale (I), où X représente CO, que l'on peut éventuellement ne pas isoler, avant de la réduire en alcool de formule générale (I), où X représente CHOH, par exemple au moyen d'un agent tel que le borohydrure de potassium.

L'alcool racémique ainsi obtenu peut ensuite être dédoublé de manière connue, par exemple par cristallisation fractionnée des sels d'addition diastéréoisomères obtenus avec un acide chiral, puis libération de la base.

Les exemples suivants illustrent la préparation des composés selon l'invention. Les microanalyses élémentaires et les spectres IR et RMN confirment les structures des produits obtenus.

## Exemple 1

(±)-1-(3,4-dihydro-2-oxo-1*H*-quinoléin-6-yl)-2-[4-(2-phényléthyl)pipéridin-1-yl]éthanol

Dans un ballon placé sous azote on introduit 3,35 g (15 mmoles) de 6-(2-chloro-1-oxoéthyl)-3,4-dihydro-1*H*-quinoléin-2-one, 3,38 g (15 mmoles) de chlorhydrate de 4-(2-phényléthyl)pipéridine, 3 g (≃30 mmoles) de carbonate de sodium, 80 ml d'éthanol et 20 ml d'eau.

On chauffe le mélange au reflux pendant 45mn, on le refroidit, on ajoute 7 g de borohydrure de potassium et on agite le mélange pendant 4h.

On ajoute 150 ml d'eau, on agite encore pendant 30mn, on filtre le précipité, on le lave à l'eau, on l'essore et on le sèche, ce qui fournit 4,1 g de produit.

On le purifie par chromatographie sur colonne de gel de silice en éluant avec un mélange 9/1 de dichlorométhane/méthanol, puis par recristallisation dans l'éthanol.

On obtient finalement 3,55 g de produit pur.

Point de fusion : 164-165°C.

Exemple 2

(±)-1-(3,4-dihydro-2-oxo-1*H*-quinoléin-6-yl)-2-[4-(2-phényléthyl)pipéridin-1-yl]éthanol, son chlorhydrate et son méthanesulfonate

Dans un ballon placé sous azote on introduit 22,3 g (100 mmoles) de 6-(2-chloro-1-oxoéthyl)-3,4-dihydro-1*H*-quinoléin2-one, 22,5 g (100 mmoles) de chlorhydrate de 4-(2-phényléthyl)pipéridine, 20 g (≃200 mmoles) de carbonate de sodium, 450 ml d'éthanol et 150 ml d'eau.
On chauffe le mélange au reflux pendant 1h, on le refroidit, on sépare le précipité (cétone de formule générale (I)) par filtration et on en prélève 6,9 g pour la purifier ultérieurement.
On réintroduit le reste de précipité dans le milieu réactionnel, on ajoute 40 g de borohydrure de potassium et on agite le mélange à température ambiante pendant 15h.
On le verse dans 900 ml d'eau, on agite pendant 30mn, on sépare le précipité par filtration, on le lave à l'eau, on le sèche et on le recristallise dans 600 ml d'éthanol.
On obtient finalement 24 g de composé.
Point de fusion : 163-164,5°C.

Pour préparer le chlorhydrate on dissout 10 g (26,4 mmoles) de base dans 400 ml de propan-2-ol au reflux et on fait barboter un courant d'acide chlorhydrique gazeux dans la solution. Après refroidissement au bain de glace on sépare le précipité par filtration, on le recristallise dans 350 ml de propan-2-ol à 1% d'acide chlorhydrique concentré et on le sèche en présence de pentoxyde de phosphore.
On obtient finalement 6,92 g de chlorhydrate.
Point de fusion : 189-190°C.

Pour préparer le méthanesulfonate on dissout 10 g (26,4 mmoles) de base dans 400 ml de propan-2-ol au reflux, on filtre à chaud et on ajoute au filtrat une solution de 2,7 g (28 mmoles) d'acide méthanesulfonique dans 10 ml de propan-2-ol. Après refroidissement au bain de glace on filtre le précipité, on le recristallise dans 200 ml d'éthanol et on le sèche en présence de pentoxyde de phosphore.
On obtient finalement 8,63 g de méthanesulfonate.
Point de fusion : 197-198°C.

Exemple 3

1-(3,4-dihydro-2-oxo-1*H*-quinoléin-6-yl)-2-[4-(2-phényléthyl)-pipéridin-1-yl]éthanone

On recristallise les 6,9 g de cétone intermédiaire séparés précédemment dans 130 ml de propanol, on lave les cristaux à l'éthanol et on les sèche.
On obtient 5,56 g de composé.
Point de fusion : 182-183°C.

Exemple 4

(-)-1-(3,4-dihydro-2-oxo-1*H*-quinoléin-6-yl)-2-[4-(2-phényléthyl)pipéridin-1-yl]éthanol

On introduit dans un erlenmeyer 20 g (52,8 mmoles) de (±)-1-(3,4-dihydro-2-oxo-1*H*-quinoléin-6-yl)-2-[4-(2-phényléthyl)pipéridin-1-yl]éthanol, 8 g (52,8 mmoles) d'acide L(+)-mandélique et 100 ml d'éthanol, et on agite le mélange, qui se prend en masse. On le chauffe au reflux en ajoutant 1100 ml d'éthanol (dissolution du sel), on filtre la solution à chaud, on réchauffe le filtrat jusqu'à nouvelle dissolution du sel, et on le laisse refroidir à température ambiante sans agitation pendant 4h.
On sépare le précipité par filtration, on le lave avec un peu d'éthanol, on le sèche, et on le recristallise deux fois dans l'éthanol. On obtient 7,21 g de sel.
Point de fusion : 216-216,5°C.
On en prélève 7,0 g qu'on traite par de l'ammoniaque dans du dichlorométhane pour en libérer la base. Après séparation de la phase organique et évaporation du solvant on recristallise le résidu dans 90 ml de propanol.
On obtient finalement 4,64 g d'énantiomère lévogyre.
Point de fusion : 184,5-185°C $[\alpha]_D^{20}$ = -45,4° (c=1 ; $CHCl_3$).

Exemple 5

(+)-1-(3,4-dihydro-2-oxo-1*H*-quinoléin-6-yl)-2-[4-(2-phényléthyl)pipéridin-1-yl]éthanol

On évapore les eaux mères issues de la cristallisation fractionnée précédente et on traite le résidu par de l'ammoniaque dans du dichlorométhane. Après séparation de la phase organique et évaporation du solvant on obtient 14,15 g (37,3 mmoles) de base qu'on met en suspension dans 100 ml d'éthanol, on ajoute 5,68 g (37,3 mmoles) d'acide D(-)-mandélique, on chauffe le mélange au reflux en ajoutant 1100 ml d'éthanol (dissolution du sel). On filtre la solution à chaud et on laisse refroidir le filtrat à température ambiante sans agitation pendant 4h.
On sépare le précipité par filtration et on le recristallise deux fois dans l'éthanol. On obtient 6,1 g de sel.
Point de fusion : 216-217°C.
On en prélève 6,0 g qu'on traite par de l'ammoniaque dans du dichlorométhane pour en libérer la base. Après séparation de la phase organique et évaporation du solvant on recristallise le résidu dans 70 ml de propanol.
On obtient finalement 3,97 g d'énantiomère dextrogyre.
Point de fusion : 185-185,5°C $[\alpha]_D^{20}$ = +43,3° (c=1 ; $CHCl_3$).

Les composés de l'invention ont fait l'objet d'essais pharmacologiques qui ont mis en évidence leur intérêt comme substances actives de médicaments.

Ainsi, leur activité neuroprotectrice a été montrée dans un modèle d'ischémie focale par ligature de l'artère cérébrale moyenne chez la souris, selon une méthode analogue à celle décrite dans *Brain Research*, **522**, (1990), 290-307.

Six jours après occlusion de l'artère cérébrale moyenne par électrocoagulation sous anesthésie à l'halothane, les souris sont à nouveau anesthésiées et le cortex cérébral ipsilatéral à l'occlusion est prélevé. Après homogénéisation du tissu, l'étendue de l'infarctus cérébral est évaluée par mesure de l'augmentation de la densité des sites benzodiazépiniques périphériques ($\omega_3$) à l'aide du composé [$^3$H]-PK 11195 de New England Nuclear.
Les traitements sont administrés curativement aux temps 5mn, 3h, 6h, 18h et 24h, par voie intrapéritonéale.

Avec les composés de l'invention, la réduction de la taille de la lésion est de l'ordre de 50 à 70%, selon le composé, à la dose de 10 mg/kg.

Les composés de l'invention ont aussi fait l'objet d'un essai d'inhibition de la liaison du [$^3$H]ifenprodil aux récepteurs sigma du cortex cérébral de rat (Schoemaker et coll., *Eur. J. Pharmacol.,* **183**, 1670, (1990)).

Le rat mâle Sprague-Dawley de 150 à 230 g est sacrifié et le cortex cérébral est homogénéisé dans 20 volumes de tampon Tris-HCl à 50mM (pH=7,4 à 25°C) glacé, au moyen d'un appareil Ultra-Turrax™ (Ikawerk) ou Polytron™ (Kinematica).
L'homogénat est lavé deux fois par centrifugation pendant 10minutes à 45000xg, le culot étant remis en suspension dans du tampon frais. Le culot final est repris dans 20 volumes du même tampon.
Un aliquote de 100 µl de cette suspension est incubé dans un volume final de 1000 µl avec 0,5 nM de [$^3$H]ifenprodil (activité spécifique : 30 à 35 Ci/mmole) pendant 30 minutes à 37°C, en l'absence ou en présence de substance compétitrice. Après incubation les membranes sont récupérées par filtration sur des filtres Whatman GF/B™ prétraités avec de la polyéthylèneimine à 0,05%, puis lavés avec deux fois 5 ml de tampon glacé.
On détermine la liaison non spécifique avec de l'ifenprodil 10µm, on analyse les données selon les méthodes usuelles, et on calcule la concentration $CI_{50}$, concentration qui inhibe de 50% la liaison du [$^3$H]ifenprodil.

Les $CI_{50}$ des composés de l'invention se situent, dans cet essai, entre 3 et 50 nM.

Les composés de l'invention ont encore fait l'objet d'un essai d'inhibition de la liaison du [$^3$H]ifenprodil aux récepteurs sensibles aux polyamines du cortex cérébral de rat, d'après le protocole décrit par Schoemaker et coll., *Eur. J. Pharmacol.,* **176**, 249-250, (1990).

Le rat mâle Sprague-Dawley de 150 à 230 g est sacrifié et le cortex cérébral est homogénéisé dans 20 volumes de tampon Tris-HCl à 50mM (pH=7,4 à 0°C) glacé, au moyen d'un appareil Ultra-Turrax™ (Ikawerk) ou Polytron™ (Kinematica).
L'homogénat est lavé deux fois par centrifugation pendant 10mn à 45000xg, le culot étant remis en suspension dans du tampon frais. Le culot final est repris dans 20 volumes du même tampon.
Un aliquote de 100 µl de cette suspension est incubé dans un volume final de 1000 µl avec 1 nM de [$^3$H]ifenprodil (activité spécifique : 30 à 35 Ci/mmole) pendant 120mn à 0°C, en présence de 3 µM de GBR 12909 (Research Biochemicals Inc., Natick, MA, USA), en l'absence ou en présence de substance compétitrice.
Après incubation, le mélange est dilué avec 5 ml de tampon Tris-HCl à 50mM (pH=7,4 à 0°C) glacé et les membranes sont récupérées par filtration sur des filtres Whatman GF/B™ prétraités avec de la polyéthylèneimine à 0,05%, puis lavés avec deux fois 5 ml de tampon glacé.

On détermine la liaison non spécifique avec l'ifenprodil 10µM, on analyse les données selon les méthodes usuelles, et on calcule la concentration $CI_{50}$, concentration qui inhibe de 50% la liaison du [$^3$H]ifenprodil.

Les $CI_{50}$ des composés de l'invention se situent, dans cet essai, entre 6 et 16 nM.

Enfin les composés de l'invention ont été étudiés quant à leurs effets vis-à-vis des convulsions maximales induites chez la souris par électrochoc supramaximal.

Le protocole de cet essai est décrit par E. A. Swinyard et J. H. Woodhead dans *Antiepileptic Drugs*, Raven Press, New York, 111-126 (1982).

30 minutes après administration intrapéritonéale du composé à tester, on note le nombre de souris présentant des convulsions (extensions des pattes arrière), immédiatement après application d'un courant électrique (0,4 s, 60 mA, 50 Hz) à l'aide d'électrodes transcornéennes. Les résultats sont exprimés par la $DA_{50}$, dose qui protège 50% des animaux, calculée selon la méthode de J. T. Lichtfield et F. Wilcoxon (*J. Pharm. Exp. Ther.*, **96**, 99-113 (1949)) à partir de 3 ou 4 doses administrées chacune à un groupe de 8 à 10 souris.

Les $DA_{50}$ des composés de l'invention se situent, dans cet essai, entre 10 et 40 mg/kg par la voie intrapéritonéale.

D'autres essais effectués *in vivo* sur la souris ont par ailleurs montré que le composé selon l'invention a des propriétés anticonvulsivantes vis-à-vis des effets du *N*-méthyl-D-aspartate (NMDA).

Les résultats des essais effectués sur les composés de l'invention suggèrent qu'ils peuvent être utilisés pour le traitement et la prévention de désordres cérébraux tels que ceux qui sont consécutifs, par exemple, à une attaque ischémique, un arrêt cardiaque ou respiratoire, une thrombose ou une embolie cérébrale, pour le traitement de la sénilité cérébrale, de la démence consécutive aux infarctus multiples, de la démence sénile, par exemple de la maladie d'Alzheimer ou de la maladie de Pick, pour le traitement de l'atrophie olivo-ponto-cérébellaire et d'autres maladies neurodégénératives telles que la chorée de Huntington, pour le traitement de la schizophrénie, pour le traitement des traumatismes crâniens ou spinaux, pour le traitement des états convulsifs, pour le traitement de certains cancers, pour le traitement du SIDA, et comme antiémétique, par exemple lors de traitements de cancers par le cisplatine.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 1 à 1000 mg de substance active.

## Revendications

1. Composé de formule générale (I)

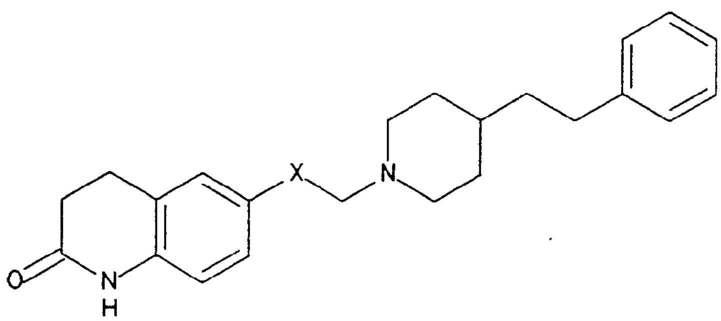

dans laquelle X représente un groupe de formule CO ou CHOH, à l'état de base libre ou de sel d'addition à un acide et, lorsque X représente un groupe de formule CHOH, sous forme d'énantiomère pur ou de mélange d'énantiomères.

2. Composé selon la revendication 1, en l'espèce le (±)-1-(3,4-dihydro-2-oxo-1*H*-quinoléin-6-yl)-2-[4-(2-phényléthyl)pipéridin-1-yl]éthanol.

3. Composé selon la revendication 1, en l'espèce le (-)-1-(3,4-dihydro-2-oxo-1*H*-quinoléin-6-yl)-2-[4-(2-phényléthyl)pipéridin-1-yl]éthanol.

4. Composé selon la revendication 1, en l'espèce le (+)-1-(3,4-dihydro-2-oxo-1*H*-quinoléin-6-yl)-2-[4-(2-phényléthyl)pipéridin-1-yl]éthanol.

5. Composé selon la revendication 1, en l'espèce la 1-(3,4-dihydro-2-oxo-1*H*-quinoléin-6-yl)-2-[4-(2-phényléthyl)-pipéridin-1-yl]éthanone.

6. Procédé de préparation d'un composé selon la revendication 1, caractérisé en ce qu'on fait réagir la 6-(2-chloro-1-oxoéthyl)-3,4-dihydro-1*H*-quinoléin-2-one avec la 4-(2-phényléthyl)pipéridine, pour obtenir d'abord la cétone de formule générale (I), où X représente CO, puis, si on le désire, on réduit cette dernière en alcool de formule générale (I), où X représente CHOH.

7. Médicament caractérisé en ce qu'il consiste en un composé selon l'une des revendications 1 à 5.

8. Composition pharmaceutique caractérisée en ce qu'elle contient un composé selon l'une des revendications 1 à 5, associé à un excipient pharmaceutique.

**Revendication pour les Etats contractants suivant : ES, GR**

1. Procédé de préparation d'un composé de formule générale (I)

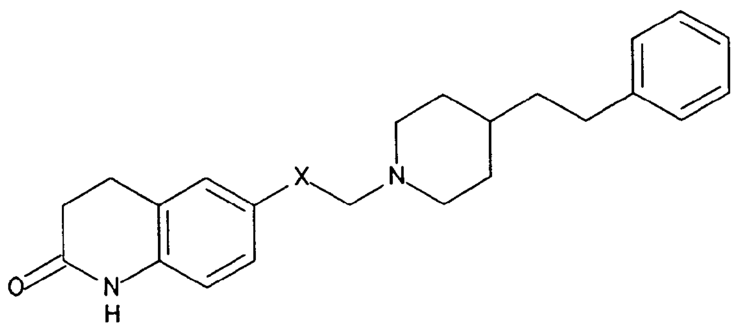

dans laquelle X représente un groupe de formule CO ou CHOH, caractérisé en ce qu'on fait réagir la 6-(2-chloro-1-oxo-éthyl)-3,4-dihydro-1*H*-quinoléin-2-one avec la 4-(2-phényléthyl)pipéridine, pour obtenir d'abord la cétone de formule générale (I), où X représente CO, puis, si on le désire, on réduit cette dernière en alcool de formule générale (I), où X représente CHOH.

Office européen des brevets

# RAPPORT DE RECHERCHE EUROPEENNE

Numero de la demande

EP 91 40 2716

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-0 351 282 (SYNTHELABO)<br>*TABLEAU*<br>--- | 1,7,8 | C07D401/06<br>A61K31/47 |
| A | GB-A-2 071 094 (OTSUKA PHARMACEUTICAL CO. LTD.)<br>* page 2, ligne 35 - page 3, ligne 12; revendication 1 *<br>----- | 1,7,8 | |

| DOMAINES TECHNIQUES RECHERCHES (Int. Cl.5 ) |
|---|
| C07D |

Le présent rapport a été établi pour toutes les revendications

| Lieu de la recherche | Date d'achèvement de la recherche | Examinateur |
|---|---|---|
| LA HAYE | 29 NOVEMBRE 1991 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)